# EUROPEAN PATENT APPLICATION

(11) **EP 2 156 801 A1**
(43) Date of publication of application: **24.02.2010**
(21) Application number: 09010670.9
(22) Date of filing: 19.08.2009
(51) Int. Cl.: A61B 18/14

(54) **High frequency surgical instrument**

(30) Priority: 20.08.2008 JP 2008211741
(71) Applicant: FUJIFILM Corporation, Minato-ku Tokyo (JP)
(72) Inventor: Akahane, Hidefumi, Saitama-shi Saitama (JP)
(74) Representative: Höhfeld, Jochen

(57) **Abstract**

An endoscopic surgical instrument of the sort having a flexible cord encased in a flexible sheath and attached to a tail end of an electrode member to be retractably protruded from a fore distal end of the flexible sheath. A fluid passage is provided internally of and throughout the flexible sheath to extend as far as a fore distal end of the latter. An influx/efflux control valve is connected to a rear proximal end of the fluid passage. The influx/efflux control valve is provided with a plural number of influent/effluent fluid connection ports in association with a switching means which is adapted to selectively connect the influent/effluent fluid connection ports with the fluid passage in the flexible sheath.

## Description

### [TECHNICAL FIELD]

This invention relates to a high frequency surgical instrument to be introduced into a body cavity by way of a tool guide channel of an endoscope, for example, for giving a surgical treatment such as mucosal dissection, lavation of an intracavitary site or coagulative stanching by the use of a high frequency knife.

### [TECHNICAL BACKGROUND]

When a lesion is spotted in a mucosal membrane on an intracavitary wall of the esophagus, stomach or colon by an endoscopic examination, the mucosal lesion is dissected by an endoscopic surgical treatment, e.g., by a treatment which is generally called ESD (Endoscopic Submucosal Dissection). In an ESD treatment, after marking and bloating up a mucosal lesion to be removed, a dissection is made along a marking line by the use of a high frequency tool, cutting off fiber of a submucosal layer to detach the mucosal lesion from the muscular tunics.

Endoscopic high frequency surgical instruments, namely, endoscopic electro-surgical dissection tools currently in use in ESD treatments are mostly in the form of a high frequency knife having a rod-like electrode member protrusibly encased in a flexible sheath. Connected to a proximal end of the flexible sheath is a manipulation means thereby to pull or push the high frequency knife into or out of the flexible sheath. At the time of mucosal dissection and excision, for example, current is conducted through the high frequency knife which is projected out of the flexible sheath. The high frequency knives of this sort are largely classified into a needle type and a hooked type. A needle type high frequency knife employs a straight needle-like electrode member while a hooked type high frequency knife employs an electrode member with a bulged electrode portion of an increased diameter or a hook portion of L-shape at a distal end of a rod-like electrode member.

The needle type knife is most suitably used in piercing the mucous tunics, and put in a horizontal movement along the mucous tunics or in a swing motion at the time of dissection or excision of the mucous tunics. On the other hand, in the case of a hooked type knife, a mucosal dissection or excision treatment is performed by catching and pulling a mucosal membrane by a hooked end portion of the knife.

In Japanese patent application laid open under 2004-313537, for instance, a high frequency surgical instrument in the form of a hooked high frequency knife is introduced into a body cavity by way of a tool guide channel of an endoscope at the time of performing a mucosal dissection or excision treatment. In this case, the high frequency knife which is encased in a flexible sheath is provided with an electrode portion of a circular or triangular shape contiguously at the distal end of a rod-like electrode member to serve as a hook portion. This high frequency surgical instrument is protrudable into a body cavity from a tool exit opening at the fore distal end of the endoscopic tool guide channel. By pushing and pulling the high frequency knife outward and inward of the protruded flexible sheath, a target mucosal lesion is caught on the hook portion at the distal end of the knife and taken into the endoscopic tool guide channel. In the course of this electro-surgical treatment, a current is conducted through the high frequency knife thereby to dissect or excise mucosal tissues.

By the way, in order to carry out excision of a mucous membrane precisely, it is necessary to capture a target mucosal lesion clearly in the view field of an endoscopic observation optics window. In some cases it becomes necessary to irrigate a mucosal portion under a surgical treatment by the use of a physiological saline solution to wash away body fluids or other smudges covering the mucosal portion. Besides, it may become necessary to supply a physiological saline solution to a bleeding spot during a surgical treatment. Since an electrode member and a power supply cable are loosely packed in a flexible sheath, interstices or gap spaces between these parts can be utilized as a liquid supply route.

Further, in an endoscopic examination or surgical operation, it often becomes necessary to aspirate an intracavitary site. Normally, aspiration is carried out by the use of a tool guide channel of an endoscope. However, it is undesirable to actuate an aspiration means while a high frequency surgical tool is still in an endoscopic tool guide channel. This is because an aspiratory passage is joined with an endoscopic tool guide channel at a junction with a tool entrance way which is provided on a manipulating head grip of the endoscope as an approach to the tool guide channel and which is adapted to receive a surgical tool in a loose state. That is to say, there is always a gap space around a surgical tool in the tool entrance way, so that, if a suction force is applied to the aspiratory passage in this state, it is very likely that leaks of suction force take place through the gap spaces around the surgical tool in the tool entrance way, in addition to outflow of aspirated body fluids through the tool entrance way. In addition, aspiration through narrow gap spaces as mentioned above usually suffers from drops in aspiratory suction force level, and thus from low performance quality.

### [SUMMARY OF THE INVENTION]

In view of the foregoing situations, it is an object of the present invention to provide a high frequency surgical instrument which is capable of pumping a liquid or fluid to and from an intracavitary site under a surgical treatment when it is inserted into a body cavity by way of a tool guide channel of an endoscope.

It is another object of the present invention to provide a high frequent surgical instrument which is switchable between a liquid feed mode and an aspiratory mode while being manipulated by way of a tool guide channel of an endoscope.

According to the present invention, for the purpose of achieving the above-stated objectives, there is provided a flexible sheath to be introduced into a tool guide channel in an endoscope, a flexible cord provided within said flexible sheath, an electrode provided at a tip side of said flexible cord adapted to be retractably protruded from a fore distal end of said flexible sheath, a fluid passage provided in said flexible sheath and opened at a fore distal end thereof, an influx/efflux control means provided in a proximal side of said fluid passage having a plural number of influent/effluent connection ports adapted to be brought into communication with said fluid passage, and a switch means for selectively connecting one of said influent/effluent connection ports to said fluid passage.

Although more than three influent/effluent fluid connection ports may be provided on the influx/efflux control valve, the connection ports should at least include a fluid feed tube connection port and an aspiratory tube connection port. That is to say, the influx/efflux control valve should be provided with a port for connecting a fluid feed tube to be used in sending a fluid into an intracavitary site under a surgical treatment, and a port for connecting an aspiratory tube to be used aspiratory purposes. The influx/efflux control valve is largely composed of a valve casing and a switching valve member which is put in a sliding displacement in an axial or radial direction within the valve casing to connect either the fluid feed tube connection port or the aspiratory tube connection port selectively with the fluid passage in the flexible sheath of the electro-surgical instrument. In this instance, normally the switching valve member is retained in an initial position by a biasing means, connecting the fluid feed tube connection port with the fluid passage in the flexible sheath. The switching valve member can be manually shifted to an aspiratory position, connecting the aspiratory tube connection port to the fluid passage in the flexible sheath. By the action of the above-mentioned biasing means, the switching valve member is automatically returned to the initial position as soon as it is relieved of a manual operating force. In shifting the position of the switching valve member within the valve casing, to and from the initial position and the aspiratory position, the valve member is put in a straight sliding displacement in the axial direction of the valve casing or in a rotary sliding displacement about the center axis of the valve casing.

A passage for a flexible cord tailed to an electrode member may be provided separately from a fluid passage within the flexible sheath in the fashion of a multi-lumen tube. However, for the sake of reduction in diameter, it is desirable to use a single passage for the flexible cord and a fluid passage to which the above-mentioned influx/efflux control valve is connected. Besides, it is desirable to provide a restraint mechanism thereby to limit a projection length of the electrode member from a fore distal end of the flexible sheath, from the standpoint of safety of surgical treatments. For this purpose, a stopper ring of an electrically insulating material is fitted in a fore distal end portion of the flexible sheath. The stopper ring internally defines an axial passage hole in communication with the fluid passage in the flexible tube, letting the electrode member to retractably protrude out of the axial passage hole to a preset projection length from a fore distal end of the flexible sheath. The axial passage hole of the stopper ring is constantly communicated with the fluid passage in the flexible sheath. A self-restraint member which is provided on the electrode member or on the flexible cord at the tail end of the electrode member is brought into abutting engagement with an inner end of the stopper ring to limit a maximum projection length of the electrode member. When the self-restraint member is abutted against the stopper ring, segmental fluid passages are formed around the electrode member in the axial passage hole of the stopper ring to maintain communication with the fluid passage in the flexible sheath.

The above and other objects, features and advantages of the invention will become apparent from the following particular description of the invention, taken in conjunction with the accompanying drawings which show preferred embodiment of the invention. Needless to say, the present invention should not be construed as being limited particular forms shown in the drawings.

### [BRIEF DESCRIPTION OF THE DRAWINGS]

In the accompanying drawings:
Fig. 1 is a schematic general view of an electro-surgical instrument embodying the present invention;
Fig. 2 is a fragmentary sectional view on an enlarged scale of the surgical instrument shown in Fig. 1;
Fig. 3 is an enlarged fragmentary sectional view of a fore end portion of the surgical instrument of Fig. 2, with an electrode member retracted into a flexible sheath;
Fig. 4 is an enlarged fragmentary sectional view of the fore end portion of the surgical instrument, with the electrode member projected out of the flexible sheath;
Fig. 5 is a schematic cross-section taken on line X-X in Fig. 4;
Fig. 6 is a diagrammatic illustration of an influent and effluent fluid feed system to be connected to the surgical instrument;
Fig. 7 is a schematic sectional view of an influx/efflux control valve unit, adopted as a first embodiment of the invention;
Fig. 8 is a fragmentary outer view of a distal end portion of an endoscopic insertion tube, having a surgical instrument protruded out of a tool guide channel in an endoscopic insertion tube;
Fig. 9 is a schematic sectional view of another influx/efflux control valve unit, adopted as a second embodiment of the invention; and
Fig. 10 is a schematic cross-section taken on line Y-Y in Fig. 9.

### [DESCRIPTION OF PREFERRED EMBODIMENTS]

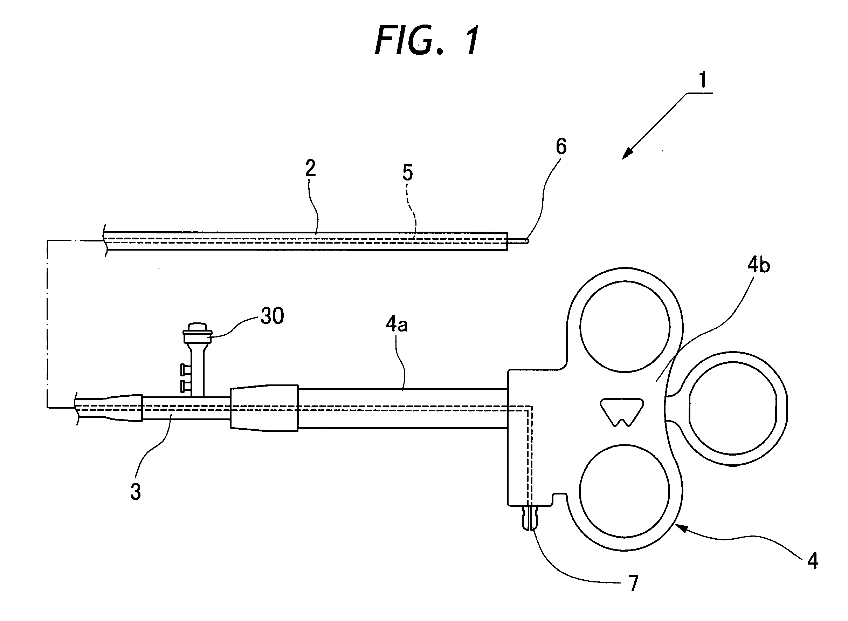

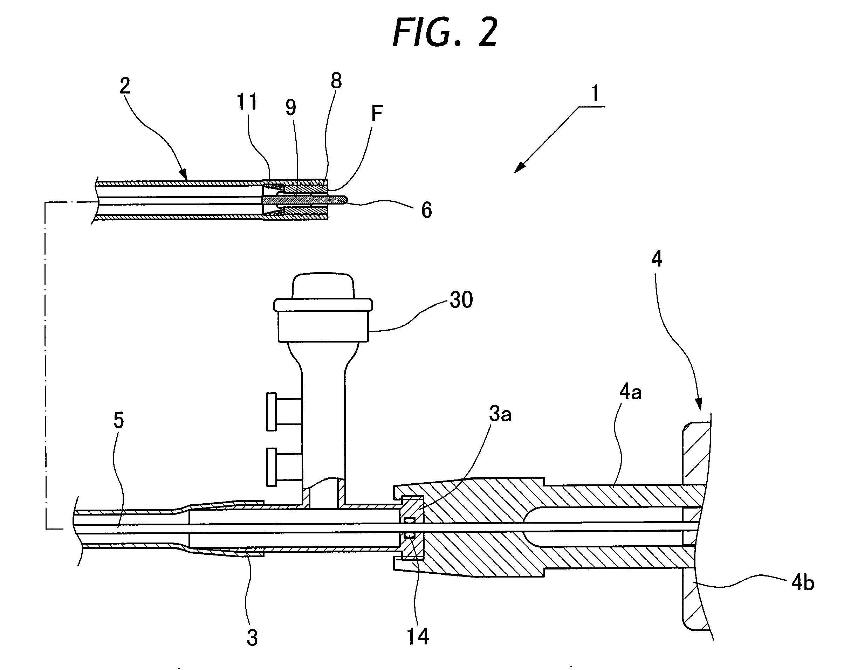

Hereafter, the invention is described more particularly by way of its preferred embodiments with reference to the accompanying drawings. Fig. 1 shows the general layout of an endoscopic high frequency electro-surgical instrument embodying the present invention, and Fig. 2 shows essential parts of the high frequency surgical instrument in an enlarged sectional view. In these figures, indicated at 1 is an endoscopic electro-surgical instrument, including an elongated flexible sheath 2 having a proximal end thereof in hermetical fitting engagement with one end of a connector pipe 3 serving as a joint member. The other end of the connector pipe 3 is hermetically connected to a manipulation means 4. The manipulation means 4 is largely composed of a shank portion 4a which is connected to the connector pipe 3, and a slider 4b which is axially slidable on and along the shank portion 4a.

Connected to the slider 4b is a proximal end of a flexible cord 5 of the electro-surgical instrument 1 which is encased in the flexible sheath 2. The flexible cord 5 is constituted by a conducting wire which is covered with an insulating fluorine resin coating or the like. A needle electrode 6 is attached to the fore distal end of the flexible cord 5 to serve as a needle type high frequency knife. On the other hand, the flexible cord 5 is provided with a contact point 7 at a proximal end which is led out to a lateral side of the slider 4b. Thus, by way of the contact point 7, the flexible cord 5 is disconnectibly connectible to a high frequency power supply which is not shown in the drawings.

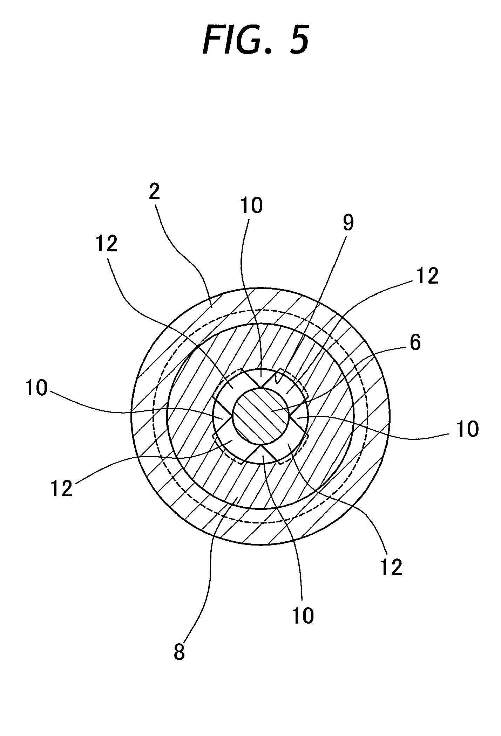

As clear from Fig. 2, the flexible cord 5 from the slider 4b is passed internally through the connector pipe 3 and the flexible sheath 2. The electrode member 6 at the fore distal end of the flexible cord 5 is retractably protrudable into and out of the flexible sheath 2. A stopper member 8 is fixedly fitted in a fore end portion of the flexible sheath 2. The stopper member 8 is formed of an insulating rigid material like ceramics, for example, and its fore end face is disposed flush with an end face of the flexible sheath 2 to provide a distal reference surface F together with the latter at the fore distal end of the surgical instrument 1. An axial passage hole 9 is bored axially through the stopper member on and along the center axis thereof. The axial passage hole 9 has an inside diameter which is appreciably large enough as compared with the thickness of the electrode member 6. Accordingly, an annular fluid passage 10 is formed between the electrode member 6 and inner wall surface of the axial passage hole 9 (Fig. 5). Further, a guide member 11 is located contiguously at the inner end of the stopper member 8. On the inner peripheral side, the guide member 11 is formed with a guide surface which is tapered off toward the stopper member 8.

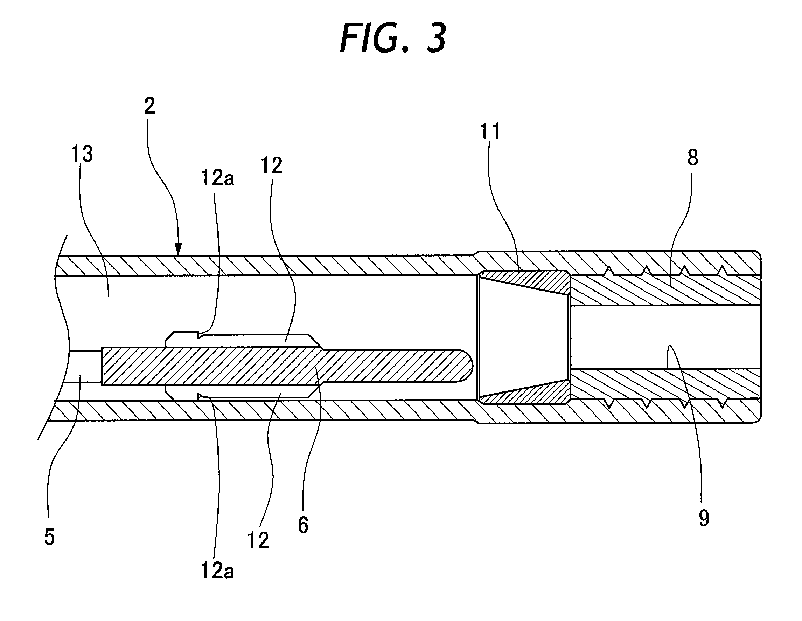

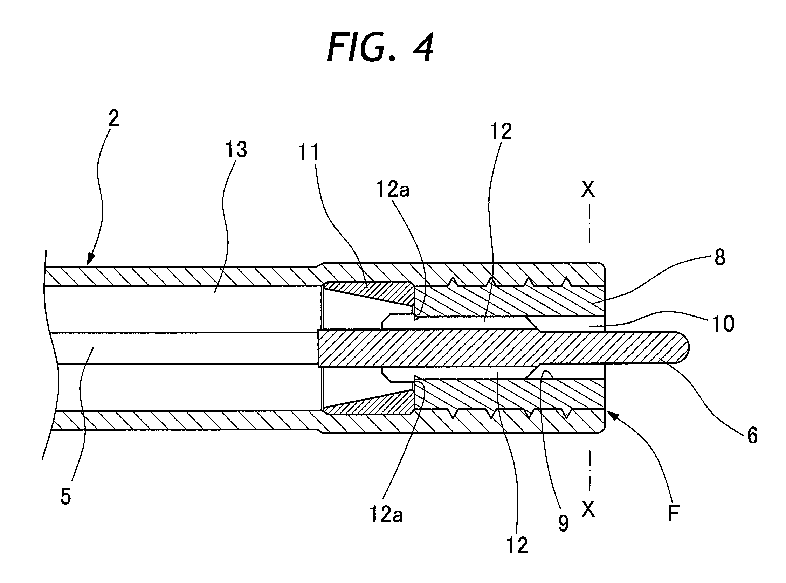

Normally, the electrode member 6 is located in a retracted position inward of the stopper member 8 as shown in Fig. 3. When the flexible cord 5 is pushed forward by way of the manipulation means 4, the electrode member 6 is moved forward through the axial passage hole 9 in the stopper member 8 to protrude from the distal end of the flexible sheath 2 to a required projection length. As shown in Figs. 4 and 5, the electrode member 6 is formed in a rod-like shape and rounded off at its fore distal end in a convex semi-spherical shape. In a rear end portion of the electrode member 6, four blade-like self-restraint members 12 are projected in radially outward directions from the electrode member 6 at intervals of 90 degrees in the circumferential direction. As the electrode member 6 is pushed into the axial passage hole 9 of the stopper member 8, outer projected ends of the self-restraint members 12 are substantially brought into sliding contact with inner surfaces of the axial passage hole 9. At the rear end, each one of the self-restraint members 12 is provided with a stopper fin portion 12a which is projected radially outward to a greater degree as compared with the remainder of the self-restraint member 12. On the other hand, the stopper member 8 is arranged to have a smaller inside diameter as compared with the minimum inside diameter at the tapered fore end of the guide member 11. Accordingly, the electrode member 8 can be pushed into the axial passage hole 9 of the stopper member 8 until the stopper fin portions 12a at the rear ends of the respective self-restraint members 12 come to abutting engagement with the inner end of the stopper member 8. That is to say, the position where the stopper fin members 12a of the respective self-restraint members 12 are abutted against the inner end of the stopper member 8 determines the maximum projection length of the electrode member 6 from the flexible sheath 2.

As described hereinlater, the electro-surgical instrument 1 is used as a high frequency knife, for example, for excision of a mucosal lesion on an intracavitary wall. For this purpose, the maximum projection length of the electrode member 6 from the annular reference surface F at the distal end of the flexible sheath 2 is set at a value which is greater than the thickness of a target mucous membrane but smaller than the sum of the thicknessof the mucous membrane and the thickness of a submucosal layer. The inside of the flexible sheath 2 functions as a fluid passage 13 at the time of pumping in or pumping out a fluid through the connector pipe 3 of the manipulation means 4, which is connected to the proximal end of the flexible sheath 2.

As shown in Fig. 3, if not used, the electrode member 6 can be retracted sufficiently into the flexible sheath 2 at the time of pumping a fluid to or from an intracavitary site, using the axial passage hole 9 of the stopper member 8 as a fluid passage. On the other hand, a fluid can be pumped in or out even when the electrode member 6 is located within the axial passage hole 9 of the stopper member 8. When the electrode member 6 is in a maximally projected position, the fluid passage 10 is divided into four segmental passages by the self-restraint members 12 on the electrode member 8. Thus, a fluid in a fluid passage 13 inside the flexible sheath 2 is spurted out through the fluid passage 10 around the electrode member 6 in the axial passage hole 9, which is divided into four segmental passages by the self-restraint members 12. Proximal end of the flexible cord 5 is passed through the connector pipe 3 and connected to the contact point 7.

In order to seal off the fluid passage 13 around the flexible cord 5 at the proximal end of the connector pipe 3 which is connected to the manipulation means 4, the connector pipe 3 is provided with a seal portion 3a which is reduced in inside diameter and adapted to hermetically fit around the flexible cord 5 through a seal ring 14.

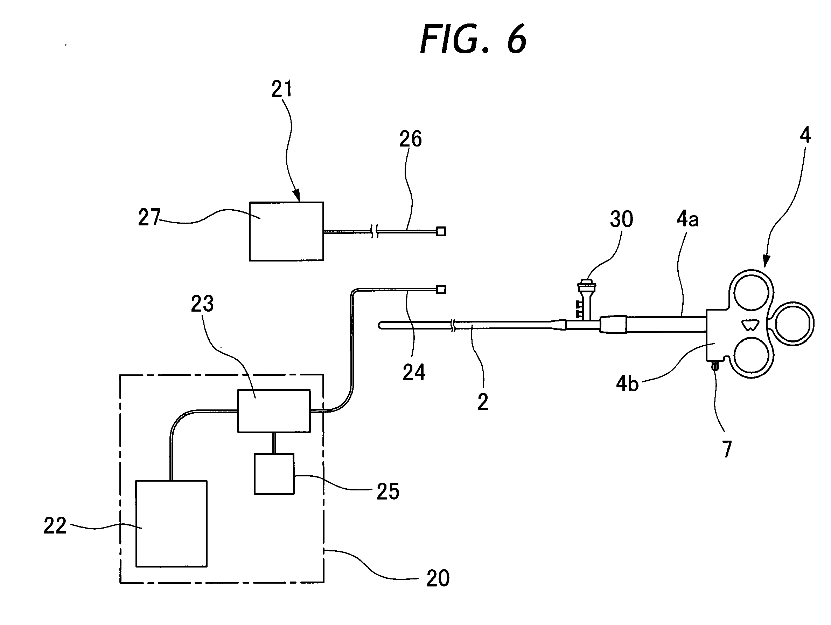

A fluid is pumped in toward an intracavitary site under treatment from the fluid passage 13 via the segmental fluid passages 13, or alternatively a fluid is aspirated from an intracavitary site toward the fluid passage 13 via the segmental fluid passages 13. For this purpose, as shown in Fig. 6, the electro-surgical instrument 1 is disconnectibly connectible to a fluid feed means 20 and an aspiration means 21 as well. The fluid feed means 20 is connected when supplying a biocompatible liquid such as a physiological saline solution, sodium hyaluronate or Glyceol. More specifically, the fluid feed means 20 includes a fluid feed tank 22, and a pump 23 an intake port of which is connected to the fluid feed tank 22. A delivery port of the pump 23 is connected to a fluid supply conduit 24. Further, the pump 23 is connected to an on-off switch in the form of a foot switch, for example. On the other hand, the aspiration means 21 is largely constituted by a suction tube 26 from an aspirator 27, which is composed of an aspiration pump and an aspirate container.

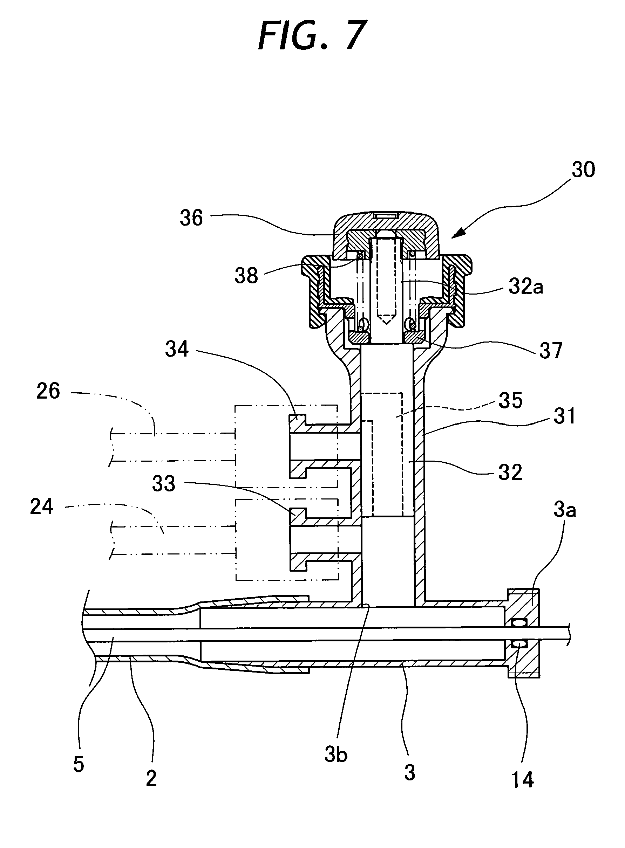

The liquid feed tube 24 and suction tube 26 are connected to a fluid influx/efflux control valve unit 30 which is shown in Fig. 7 as a first embodiment of the invention. This fluid influx/efflux control valve unit 30 is connected to the connector pipe 3 by way of a valve casing 31 which is joined with the connector pipe 3. A valve member 32 is axially reciprocally fitted in the valve casing 31.
Provided on the front side of the valve casing 31 are a liquid feed tube connection port 33 to which the liquid feed tube 24 is connectible and an aspiratory suction tube connection port 34 to which the suction tube 26 is connectible. The switch valve unit 32 is arranged to selectively connect the liquid feed tube connection port 33 or the suction tube connection port 34 to an inlet/outlet port 3b which is in communication with the fluid passage 13 in the connector pipe 3. In an initial position shown in Fig. 7, the liquid feed tube connection port 33 is connected to the outlet/inlet port 3 of the connector pipe 3. As the valve member 32 is displaced downward from the initial position of Fig. 7, the suction tube connection port 34 is brought into communication with the outlet/inlet port 3a of the connector pipe 3 through a switching passage 35 which is provided internally of the valve member 32. In this state, the liquid feed tube connection port 33 is covered and blocked by the valve member 32, out of communication with the outlet/inlet port 3a of the connector pipe 3.

A manual valve switcher is mounted on top of the valve casing 31 thereby to shift the valve member 32 between the two positions mentioned above. More specifically, a switch button 36 is fitted in an upper end of a link column 32a which is projected upward from the valve member 32. A biasing coil spring 38 is interposed in a compressed state between the switch button 36 and a spring retainer 37 which is fitted in an upper end portion of the valve casing 31. Normally, under the influence of the biasing action of the spring 38, the valve member 32 is retained in a lifted initial position. In this initial position, the liquid feed tube connection port 33 is communicated with the inlet/outlet port 3b at the lower end of the valve casing 31. When the switch button 36 is pushed in against the action of the biasing spring 38, the valve member 32 is displaced downward within the valve casing 31 and as a result shifted to an inner aspiratory position connecting the suction tube connection port 34 with the inlet/outlet port 3b.

By the use of the electro-surgical instrument 1 of the above-described construction, an ESD can be carried out to remove a submucosal layer between a mucous membrane and muscular tunics. For this purpose, in the first place, a liquid feed tube 24 and a suction tube 26 are connected to the liquid feed tube connection port 33 and suction tube connection port 34 on the valve casing 31 of the fluid influx/efflux control valve unit 30, respectively.

For a mucosal dissection and excision treatment, the projection length of the electrode member 6 out of the flexible sheath 2, i.e., a projection length from the distal reference surface F which is held in abutting engagement with the surface of a mucous membrane to be dissected, is adjusted to a value which is greater than the thickness of the mucous membrane but small enough to prevent the tip end of the electrode member 6 from reaching the underlying muscular tunics. By so doing, when the electrode member 6 is projected to a maximum projection length from the flexible sheath 2, having the distal reference surface F in abutting engagement with a surface of a dissecting mucous membrane, the electrode member 6 is driven securely to a target depth, piercing through the mucous membrane but falling short of the muscular tunics. The electrode member 6 is propelled or retracted by way of the manipulation means 4 at the proximal end of the electro-surgical instrument 1.

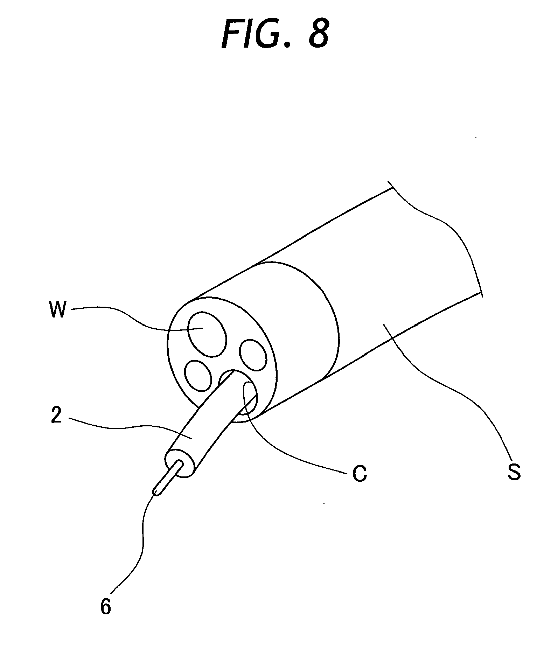

As shown in Fig. 8, the electro-surgical instrument 1 is introduced into a body cavity through a tool guide channel C in an endoscopic insertion tube S with an endoscopic observation optics window W. When a mucosal lesion is found on an intracavitary wall in an organ such as the esophagus, stomach, duodenum or large intestine, the mucosal lesion is removed by ESD. In an ESD operation, a high frequency current is conducted across the electrode member and a counter electrode plate which is put in a confronting position on the body of an examinee.

For example, a mucosal lesion is removed by an ESD operation in the manner as follows. An ESD operation of this sort is carried out when an existence of a mucosal lesion is confirmed by an endoscopic examination.

The liquid feed tube 24 is connected to the liquid tube connection port 33 on the valve casing 30, in addition to the suction tube 26 which is connected to the other suction tube connection port 34. However, when the valve member 32 is in the initial position shown in Fig. 7, the suction tube is in a blocked state and thus unable to apply a suction force to the fluid passage 13. Further, in this position, no liquid is supplied to the liquid feed tube 24 until the pump 23 is actuated by depressing the switch 25. That is to say, no fluid is fed to or aspirated from a body cavity until a fluid intake or outtake operation is started by manually pushing a start button or buttons.

The endoscopic electro-surgical instrument 1 is introduced into a body cavity by way of a tool guide channel C which is provided internally of an elongated insertion tube of an endoscope, bringing the endoscopic observation optics window to a confronting position relative to a target mucosal lesion on an intracavitary wall. Then, markings are put on around the boundaries of the mucosal lesion before swelling and uplifting the lesion by a local injection, which is followed by dissection and excision of the mucosal lesion and other necessary treatments. Throughout this ESD operation, a clear view of the intracavitary site under treatment has to be acquired through the endoscopic observation optics window W. In case a bleeding spot is detected through the endoscopic observation optics prior to proceeding to mucosal dissection, it becomes necessary to wash and stanch the bleeding spot. Further, in case an intracavitary site under treatment is smudged with body fluids, it may become necessary to rinse out the body fluids by a lavage or irrigation.

In order to wash out a bleeding spot by supply of a lavage solution, for example, while pointing the fore distal end of the flexible sheath toward a bleeding spot, the liquid feed pump 23 is turned on by depressing the control switch 25. Whereupon, a lavage solution is fed under pressure to the liquid feed tube connection port 23 from the liquid feed tube 24. Since the valve member 32 is in communication with the fluid passage 13 through the inlet/outlet port 3b, the lavage solution is fed to the fluid passage 13 in the flexible sheath 2 from the liquid feed pump 23. Then, through the axial passage hole 9 of the stopper member 8 at the fore distal end of the fluid passage 13, the lavage solution is spurted out toward the bleeding spot of the intracavitary site under a surgical treatment.

After a lavage of a bleeding spot, the operator's foot is put off the control switch 25. Whereupon, the liquid feed pump 23 is stopped to suspend the liquid feed. In this instance, in concurrence with the lavage of a bleeding spot, cauterization of a lesion may be carried out by conducting current through the electrode member 6. In that case, the electrode member 6 is retained in the projected position at the fore distal end of the flexible sheath 2 while supplying a lavage solution therethrough. Even though the electrode member 6 is retained in the axial passage hole 9 of the stopper member 8, a liquid supply is feasible through a plural number of segmental fluid passages 10 which are formed around the electrode member 6.

When a cauterization treatment is carried out continually on a lesion, the cutting quality of the electrode 6 as a knife may be degraded by deposition of carbides on outer surfaces of the electrode 6. In such a case, it becomes necessary to clean the electrode member 6. Such carbide deposits can be washed away from electrode surfaces by spurting a cleaning liquid toward a fore end portion of the electrode member 6 which is projected out of the flexible sheath 2.

If a liquid is left undrained on an intracavitary site in the course of an endoscopic surgical operation, it may block the view field of the endoscopic observation optics window W on the distal end of the endoscopic insertion tube S, affecting maneuverability of the endoscopic insertion tube and the electro-surgical instrument as well. Therefore, there may arise a necessity for aspiration of a pooled liquid in the course of an ESD operation. In such a case, the switch button 36 is pushed in to shift the position of the valve member 31 in the valve casing 31. As a consequence, the influx/efflux control valve unit 30 is switched to an aspiratory position, connecting the suction tube connection port 34 to the fluid passage 13 by way of the change-over passage 35 and through the inlet/outlet port 3b, while blocking the liquid feed tube connection port 33 with the valve member 3, bringing the liquid feed tube connection port 33 out of communication with the inlet/outlet port 3b. From an suction tube 36 of an aspirator 27, which is connected to the suction tube connection port 34, vacuum force is applied to the fluid passage 13 to aspirate liquids or fluids from an intracavitary site.

In order to carry out an aspiratory operation in a smooth and efficient manner, it is desirable to retract the electrode member 6 into a position behind the stopper member 8, leaving the axial passage hole 9 in a fully opened state. However, even if the electrode member 6 is retained in a projected position forward of the stopper member 8, an aspiratory operation is feasible through the segmental fluid passages 10 around the electrode member 6. An aspiratory operation can be ended by taking a depressing force off the switch button 36, allowing the valve member 32 to displace upward toward the initial position under the influence of the biasing force of the spring 38. As a result, the communication between the suction tube connection port 34 and the fluid passage 13 through the inlet/outlet port 3b is blocked by the valve member 32. In this initial position, the liquid feed tube connection port 33 is communicated with the fluid passage 13 but no liquid is supplied to the latter unless the liquid feed pump 23 is turned on.

In this manner, upon turning on the control switch 25, a liquid is supplied to an intracavitary site from the distal end of the flexible sheath 2. When the switch button 36 is depressed, body fluids can be sucked in through the fore distal end of the flexible sheath 2. The supply of a liquid or aspiration of fluids is feasible no matter whether the electrode member 6 is projected from the fore distal end of the flexible sheath 2 or retracted into a position behind the guide member 11. That is to say, it becomes possible to carry out various operations easily in a simplified manner, including a lavage of a bleeding spot, lift-up of a dissected portion, aspiration and cleaning of the electrode member 6.

Further, after feeding a transparent liquid into an intracavitary site, a contaminated waste liquid can be collected by the influx/efflux control valve unit 30, permitting to give an irrigative cleaning treatment smoothly in a facilitated fashion. Namely, for this purpose, while holding the switching valve member 32 in the initial position, the control switch 25 is turned on to supply a fresh liquid into a body cavity As soon as the liquid supply reaches a predetermined amount, the control switch 25 is turned off to suspend the liquid supply, followed by depression of the switch button 36 to shift the position of the valve member 32. As a result, the liquid supply is stopped, and the valve member 32 is now switched to the aspiratory position to start aspiration from an intracavitary site. By repeating the liquid supply and aspiration for a plural number of times, the intracavitary site can be cleaned to a sufficient degree to get clear picture images of the intracavitary site through the endoscopic observation optics window W.

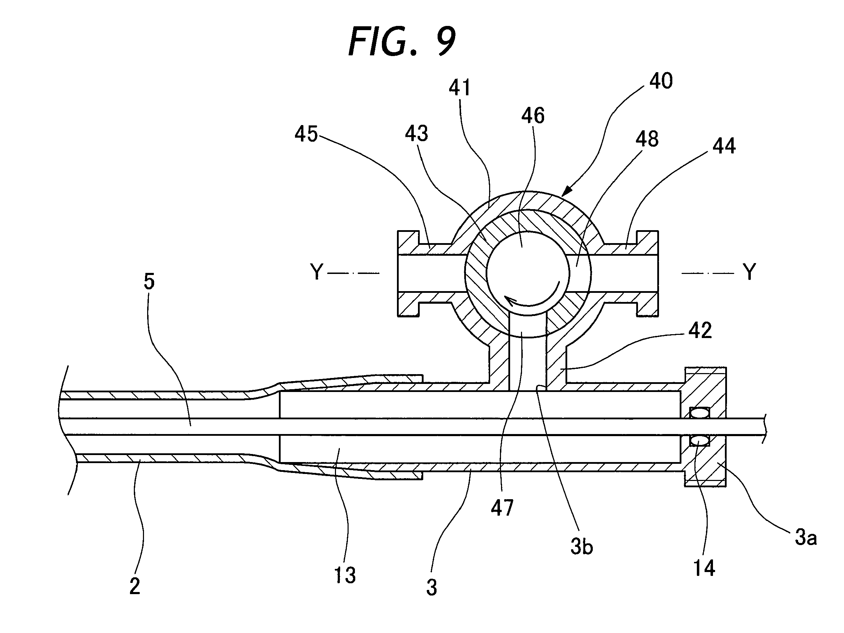

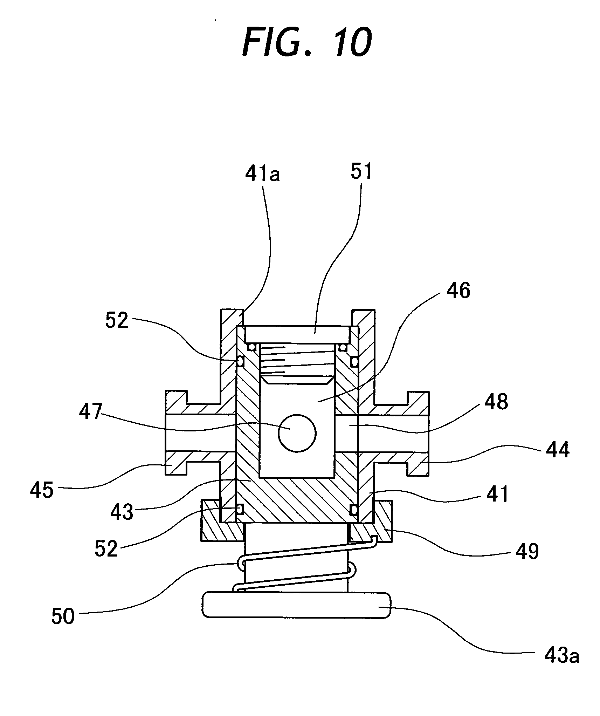

Now, turning to Figs. 9 and 10, there is shown a second embodiment of the present invention. In this embodiment, an influx/efflux control valve 40, to be connected to the connector pipe 3, is built of a cylindrical main valve casing 41 which is formed integrally with an intercommunicating pipe portion 42, which intercommunicating pipe portion42 being communicated with the connector pipe 3 through an inlet/outlet port 3b. Slidably received in the main valve casing 41 is a rotary valve member 43 for revolving movements about the axis of the valve casing 41 to and from two positions as described below.

Provided on the valve casing 41 is a liquid feed tube connection port 44 which is connectible to a liquid feed tube 24, along with a suction tube connection port 45 which is connectible to a suction tube 26. The rotary valve member 43 is switchable to and from an initial position holding the liquid feed tube connection port 44 in communication with the inlet/outlet port 3b of the connector pipe 3 through the intercommunicating pipe portion 43, and a switched aspiratory position holding the suction tube connection port 45 in communication with the inlet/outlet port 3b. A rotary knob 43a which is attached to one end of the valve member 43 can be manually turned to switch the position of the valve member 43. Further, the valve member 43 is internally provided with a switching chamber 46, in communication with first and second switch ports 47 and 48 which can be brought into communication with the suction tube connection port 45 and the liquid feed tube connection port 44, respectively, by switching back and forth the position of the valve member 43 by the switching knob 43a.

In order to retain the valve member 43 in a predetermined axial position within the valve casing 41, a stopper flange 41a is provided at one end of the valve casing 41 in such a way as to confine the valve member 43 in a predetermined axial position within the valve casing 41. A box nut 49 is threaded on the other end of the valve casing 41, i.e., on the opposite end away from the flange 41a. Thus, the valve member 43 can be immovably fixed in the axial direction by tightening the box nut. A torsion coil spring 50 is interposed between the box nut 49 and the rotary knob 43a of the valve member 43, thereby fixing the valve member 43 in an initial position. The valve member 43 can be switched to an aspiratory position by turning the rotary knob 43a through a predetermined angle in the direction of an arrow in Fig. 9, that is, in a direction contrary to the biasing action of the torsion coil spring 50. In Fig. 10, indicated at 51 is a closure cap which closes one end of the switching chamber 46, and at 52 a seal member which is interposed between the valve member 43 and the valve casing 41.

Normally, the valve member 43 is located in the initial position under the influence of the basing action of the torsion coil spring 50, connecting the first switching port 47 with the intercommunicating pipe portion 42 leading to the inlet/outlet port 3a for communication with the fluid passage 13 in the connector pipe 3, while connecting the second switching port 48 to the liquid feed tube connection port 44 through the switching chamber 46. Upon turning the rotary knob 43a to turn the valve member 43 counterclockwise as indicated by an arrow in Fig. 9, the valve member 43 is switched to an aspiratory position, connecting the first switching port 47 to the suction tube connection port 45 while connecting the second switching port 48 with the intercommunicating pipe 42.

In this instance, the valve member 43 is biased to return to the initial position by the action of the torsion coil spring 50, so that aspiration is continued only for a time period over which the rotary knob 43a is manually held in the aspiratory position. That is to say, as soon as a manual operating force is removed from the rotary knob 43a, the valve member 43 is returned to the initial position by the torsion coil spring 50. In the initial position, however, no liquid is supplied to the fluid passage 13 of the connector pipe 3 unless the control switch 25 is manually closed to start a liquid supply.

Thus, similarly to the first embodiment described above, the second embodiment of the invention makes it possible to carry out various endoscopic surgical treatments smoothly in an extremely facilitated manner, including lavage of a bleeding spot, lift-up of a dissected portion, aspiration, and cleaning of the electrode member 6 of the electro-surgical instrument.

## Claims

1. A high frequency surgical instrument which comprises:
a flexible sheath to be introduced into a tool guide channel in an endoscope;
a flexible cord provided within said flexible sheath;
an electrode provided at a tip side of said flexible cord adapted to be retractably protruded from a fore distal end of said flexible sheath;
a fluid passage provided in said flexible sheath and opened at a fore distal end thereof;
an influx/efflux control means provided in a proximal side of said fluid passage having a plural number of influent/effluent connection ports adapted to be brought into communication with said fluid passage; and
a switch means for selectively connecting one of said influent/effluent connection ports to said fluid passage.

2. A high frequency surgical instrument as set forth in claim 1, wherein plural influent/effluent connection ports comprise a fluid feed tube connection port and an aspiratory suction tube connection port;
said influx/efflux control means having a valve casing; and
said switch means consisting of a switching valve member displaceably to bring either said fluid feed tube connection port or said aspiratory tube connection port selectively into communication with said fluid passage.

3. A high frequency surgical instrument as set forth in claim 2, wherein said switching means having a urging means to urge said switching valve member for communicating between said fluid passage and said fluid feed tube connection port.

4. A high frequency surgical instrument as set forth in claim 3, wherein said switching valve member movable in an axial or radial direction in said valve casing.

5. A high frequency surgical instrument as set forth in any one of claims 1 to 4, wherein said flexible cord connected from said electrode member is axially passed through said fluid passage of said flexible sheath.

6. A high frequency surgical instrument as set forth in claim 5, wherein said flexible sheath comprising a stopper member protruded inner-wardly of the fore side of said flexible sheath;
either said flexible cord or said electrode having a restraint member to be brought into and out of abutting engagement with said stopper; and
a fluid passage being arranged in said flexible sheath when said restraint member being abutted against said stopper member.
